(19) Europäisches Patentamt
European Patent Office
Office européen des brevets

(11) Veröffentlichungsnummer: **0 043 933**
**B1**

(12) # EUROPÄISCHE PATENTSCHRIFT

(45) Veröffentlichungstag der Patentschrift: **30.05.84**

(51) Int. Cl.³: **C 07 C 85/24,** C 07 C 87/50

(21) Anmeldenummer: **81104689.5**

(22) Anmeldetag: **19.06.81**

(54) **Verfahren zur Herstellung von Polyamingemischen mit einem hohen Anteil an 4,4'-Diaminodiphenylmethan.**

(30) Priorität: **12.07.80 DE 3026554**

(43) Veröffentlichungstag der Anmeldung:
**20.01.82 Patentblatt 82/3**

(45) Bekanntmachung des Hinweises auf die Patenterteilung:
**30.05.84 Patentblatt 84/22**

(84) Benannte Vertragsstaaten:
**BE CH DE FR GB LI**

(56) Entgegenhaltungen:
**EP - A - 0 000 778**
**DD - A - 109 615**
**DE - A - 2 037 550**
**FR - A - 1 377 734**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Nestler, Gerhard, Dr., Van-Leyden-Strasse 17, D-6700 Ludwigshafen (DE)**

**EP 0 043 933 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Polyamingemischen mit einem hohen Anteil an 4,4'-Diaminodiphenylmethan durch Umsetzung von aromatischen Aminen mit Formaldehyd in Gegenwart von Styrol-divinylbenzolcopolymerisaten mit einem vernetzten Anteil zwischen 4 und 14 Gew.-% Divinylbenzol, bezogen auf das Copolymerisat, einer Korngrösse von 10 bis 200 Mikrometer mit einer spezifischen Oberfläche von 2 bis 40 Quadratmeter/Gramm und einer Porenweite von 0,5 bis 40 Nanometern.

In der europäischen Patentanmeldung 778 werden die Durchführung und Schwierigkeiten der Herstellung von Polyaminen der Diphenylmethanreihe beschrieben; sie entstehen als Kondensationsprodukte aus Anilin und Formaldehyd an sauren Katalysatoren. Zunächst reagieren Anilin und Formaldehyd auch ohne Katalysatoren zu N-Alkylverbindungen, die als Vorkondensat bezeichnet werden. Dieses Vorkondensat lagert in Gegenwart von sauren Katalysatoren in Polyamine der Diphenylmethanreihe um. Dabei entstehen eine Mischung aus 2,2'-, 2,4'-, 4,4'-Diaminodiphenylmethan und höheren Kondensationsprodukten (Dreikern- bis Sechskernverbindungen). Durch Umsetzen dieser Verbindungen mit Phosgen erhält man die entsprechenden Polyisocyanate der Diphenylmethanreihe, die wertvolle Ausgangsmaterialien bei der Herstellung von Polyurethankunststoffen, insbesondere Polyurethanschaumstoffen, darstellen. Für viele Anwendungsgebiete dieser Polyisocyanatgemische ist es von grossem Interesse, dass der in ihnen vorliegende Anteil an ortho-ständigen Isocyanatgruppen, insbesondere der Anteil an 2,2'- und 2,4'-Diisocyanatodiphenylmethan, möglichst niedrig ist. Dementsprechend strebt man bereits bei der Anilin/Formaldehyd-Kondensation Polyamingemische der Diphenylmethanreihe an, die diesen Voraussetzungen bezüglich der Isomerenverteilung entsprechen. Bei Verwendung von grossen Mengen an Salzsäure als Katalysator ist es zwar möglich, den Gehalt an o-Isomeren im Kondensationsgemisch niedrig zu halten, jedoch ist die Verwendung von Salzsäure (bzw. von Anilinhydrochlorid) mit den Nachteilen der Korrosivität sowie der Notwendigkeit einer Neutralisation bzw. aufwendigen Abtrennung des Katalysators vom Reaktionsprodukt oder der Notwendigkeit einer Abtrennung und Rückführung der Säure, wie es auch die DE-AS 22 38 920 zeigt, verbunden. Die EP-Anmeldung weist darauf hin, dass diese Nachteile zwar im Prinzip durch den Einsatz heterogener saurer Katalysatoren, z.B. Ionenaustauscher, vermieden werden können, doch ist es bei Verwendung dieser Katalysatoren bisher nicht möglich gewesen, Polyamine der Diphenylmethanreihe herzustellen, die einen Gehalt von weniger als 12% an 2,4'- und weniger als 2% an 2,2'-Diaminodiphenylmethan aufweisen.

In der EP-Anmeldung wird ein Verfahren zur Herstellung von Polyaminen der Diphenylmethanreihe mit einem hohen Gehalt an 4,4'Isomeren durch Umsetzung von aromatischen Aminen (Anilin) mit Formaldehyd in Gegenwart von sauren Ionenaustauschern beschrieben, wobei man

a) als Katalysator wassergesättigte, Sulfonsäuregruppen aufweisende, gelförmige Ionenaustauscher auf Basis von Styrol-divinylbenzol-copolymerisaten, welche mit maximal 2 Gew.-% bezogen auf Copolymerisat an Divinylbenzol vernetzt sind, oder wassergesättigte, Sulfonsäuregruppen aufweisende, makroporöse Ionenaustauscher auf Basis von Styrol-divinylbenzol-copolymerisaten, die mit mindestens 18 Gew.-% bezogen auf Copolymerisat an Divinylbenzol vernetzt sind, verwendet und

b) man die katalytische Umsetzung bei erhöhter Temperatur unter solchen Druck- und Temperaturbedingungen durchführt, dass ein Abdestillieren des Kondensationswassers und des in das System eingebrachten Wassers weitgehend unterbleibt.

Wie die Beispiele zeigen, werden Polyamine mit einem Gehalt von 73,2 bis 75,3 Gew.-% an 4,4'-Diaminodiphenylmethan, von 0,6 Gew.-% 2,2'-Diaminodiphenylmethan und von 8 bis 10,8 Gew.-% an 2,4'Diaminodiphenylmethan, bezogen auf das gesamte Polyamingemisch, erhalten. Das entspricht einem 4,4'-Isomerengehalt von höchstens 88 Gew.-% bezogen auf den Gesamtdiaminodiphenylmethangehalt.

Ältere Verfahren der Polyaminherstellung weisen die schon in der europäischen Patentanmeldung genannten Nachteile auf: so lehrt die US-PS 3 971 829 die Verwendung von Aluminiumsilikat-Oxalsäure-Mischkatalysatoren und liefert ein Polyamingemisch der Diphenylmethanreihe, dessen Anteil an Gesamtaminodiphenylmethan einen 4,4'-Isomerengehalt von nur 66 bis 77 Gew.-% aufweist.

In der DE-OS 2 037 550 wird die Kondensation von Anilinen mit wässrigem Formaldehyd in Gegenwart von sauren Ionenaustauschern beschrieben. Wie die Ausführungsbeispiele zeigen, war es durch Variation der Reaktionstemperatur und des Reaktantenverhältnisses nicht möglich, den Gehalt an 4,4'-Diaminodiphenylmethan auf 90% und mehr zu erhöhen. Auch die kontinuierliche destillative Abtrennung des Kondensationswassers während der Umsetzung brachte keine Verbesserung.

Das DD-Wirtschaftspatent 109 615 beschreibt die Bildung von Polyaminen der Diphenylmethanreihe mit einem hohen 2,4'-Diaminodiphenylmethan-Anteil durch Umsetzung von wasserfreiem Formaldehyd-Anilin-Vorkondensat in Gegenwart von sauren Ionenaustauschern auf der Basis des Copolymeren aus Styrol und Divinylbenzol, wobei der Gehalt an Divinylbenzol 1 bis 8 Gew.-% beträgt. Das Verfahren wird so durchgeführt, dass zunächst aus Anilin und Formaldehyd das Vorkondensat hergestellt wird, dieses entwässert wird und anschliessend das wasserfreie Reaktionsgemisch in Gegenwart des Ionenaustauschers bei erhöhter Temperatur umgesetzt wird. Entsprechend den Beispielen enthalten die Endprodukte 24,4 bis 26 Gew.-% 2,4'-Diaminodiphenylmethan und 34 bis 39,8 Gew.-% 4,4'-Isomer.

Auch die DE-AS 1 179 945 und die DE-PS 1 210 872 beschreiben die Kondensation von Anilin bzw. aromatischen Aminen mit wässrigem Formaldehyd in Gegenwart von sauren Ionenaustauscherharzen, wobei, wie die Beispiele zeigen, Wasser in der Regel durch Destillation entfernt wird. Die Ausführungsbeispiele zeigen zwar hohe Ausbeuten an 4,4'-Isomeren, jedoch wird in diesen Beispielen keine Angabe über den Gehalt an 2,2'- und 2,4' Isomeren gemacht. Wie das Vergleichsbeispiel 5 der EP-Anmeldung 778 zeigt, wird bei diesem Verfahren mit destillativer Entfernung des Wassers während der Kondensationsreaktion ein Polyamingemisch mit einem hohen Anteil an 2,2'- und 2,4'-Isomeren erhalten. Ausserdem weisen die angegebenen Siedeintervalle, z.B. 210–215 °C im Falle von 4,4'Diaminodiphenylmethan, auf ein Isomerengemisch hin (2,4'-Diaminodiphenylmethan:

Kp. 222°C/9 mbar; 4,4'-Diaminodiphenylmethan:

Kp. 232°C/9 mbar). Als Katalysatoren werden bevorzugt Styrol-Divinylbenzol-Copolymerisate mit einem Anteil von 1 bis 8 Gew.-%, insbesondere 2 bis 4 Gew.-% Divinylbenzol verwendet.

Es wurde nun gefunden, dass man Polyamingemische mit einem Anteil von mindestens 50 Gew.-% Diaminodiphenylmethanen, bezogen auf das Gemisch, und einem Anteil von mindestens 90 Gew.-% 4,4'-Diaminodiphenylmethan der Formel I

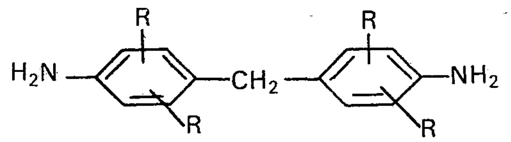
I,

worin die einzelnen Reste R gleich oder verschieden sein können und jeweils ein Wasserstoffatom, ein Halogenatom oder einen aliphatischen Rest bezeichnen, bezogen auf die Gesamtmenge an Diaminodiphenylmethanen, durch Umsetzung von aromatischen Aminen mit Formaldehyd in Gegenwart von sulfonsauren Kationenaustauschern auf Basis Styrol-divinylbenzol-copolymerisaten vorteilhaft erhält, wenn man aromatische Amine der Formel II

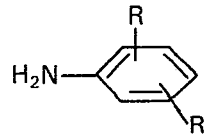
II,

worin R die vorgenannte Bedeutung besitzt, in Gegenwart von Styrol-divinylbenzol-copolymerisaten mit einem vernetzten Anteil zwischen 4 und 14 Gew.-% Divinylbenzol, bezogen auf das Copolymerisat, einer Korngrösse von 10 bis 200 Mikrometer mit einer spezifischen Oberfläche von 2 bis 40 Quadratmeter/Gramm und mit einer Porenweite von 0,5 bis 40 Nanometern umsetzt.

Die Umsetzung kann für den Fall der Verwendung von Anilin durch die folgenden Formeln wiedergegeben werden:

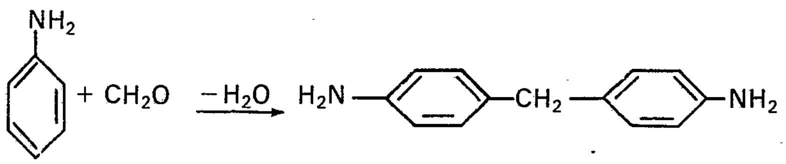


Im Vergleich zu den bekannten Verfahren liefert das Verfahren nach der Erfindung überraschend auf technisch einfacherem und wirtschaftlicherem Wege ein Polyamingemisch mit einem höheren Anteil an 4,4'-Diaminodiphenylmethan und geringerem Anteil an den 2,2'- und 2,4'-Isomeren und somit in besserer Reinheit, bezogen auf die Gesamtmenge an Diaminodiphenylmethanen, gerade auch im grosstechnischen Massstab. Die Rückführung von Teilen des Reaktionsgemisches wird vermieden. Eine aufwendige Trennung des Gemisches von den 2,2'- und 2,4'-Isomeren in einer weiteren Verfahrensstufe sind für viele Synthesen nicht notwendig. Da teilweise niedrigere Reaktionstemperaturen verwendet werden können, ist auch die Lebensdauer des Katalysators höher und somit die Wirtschaftlichkeit besser. Man erhält mindestens 50 Gew.-% zweckmässig 50 bis 90, insbesondere 60 bis 80 Gew.-% Diaminodiphenylmethane, bezogen auf das Polyamingemisch, und einen Anteil von mindestens 90 Gew.-%, insbesondere 90 bis 94 Gew.-% 4,4'-Diaminodiphenylmethan, bezogen auf die Gesamtmenge an Diaminodiphenylmethanen. Es können nicht nur makroretikulare, sondern vorteilhaft gelartige Austauscherharze verwendet werden. Bezüglich der makroretikularen bzw. gelartigen Struktur von Harzen wird auf die deutsche Patentschrift 1 168 908 verwiesen. Alle diese Vorteile des erfindungsgemässen Verfahrens sind überraschend, insbesondere auch im Hinblick auf die Lehre der europäischen Patentanmeldung. Man hätte gerade mit den erfindungsgemässen und noch mehr mit den erfindungsgemässen gelartigen Katalysatoren eine stärkere Bildung der 2-Isomeren und somit eine wesentlich geringere Ausbeute an Endstoff I erwartet. Im Gegensatz zu der Lehre der europäischen Patentanmeldung ist bei dem erfindungsgemässen Verfahren überraschend die Anwesenheit von Wasser bei der Umsetzung nicht notwendig. Daher kann im Gegensatz zu dem bekannten Verfahren die Umsetzung bei höheren Temperaturen vorteilhaft bei Normaldruck durchgeführt werden, da das Abdestillieren von Wasser während der Umsetzung nicht verhindert werden muss.

Der Formaldehyd kann in Form von Formaldehyd unter den Reaktionsbedingungen abspaltenden Stoffen, z.B. Paraformaldehyd oder Trioxymethylen, gasförmig oder vorteilhaft in Form seiner wässrigen, zweckmässig 30 bis 40 gew.-%igen Lösung, verwendet werden. Der Ausgangsstoff II kann mit Formaldehyd in stöchiometrischer Menge oder im Überschuss, vorzugsweise in einem Verhältnis von 2 bis 20, vorteilhaft 2 bis 10, insbesondere 2 bis 6 Mol Ausgangsstoff II je Mol Formaldehyd, umgesetzt werden. Bevorzugte Ausgangsstoffe II und dementsprechend bevorzugte Endstoffe I sind solche, in deren Formeln die einzelnen Reste gleich oder verschieden sein können und jeweils ein Wasserstoffatom, Chlor, Brom oder einen Alkylrest mit 1 bis 9, insbesondere 1 bis 4 Kohlenstoffatomen bedeuten. Die vorgenannten Reste können noch durch unter den Reaktionsbedingungen inerte Gruppen, z.B. Alkylgruppen mit 1 bis 3 Kohlenstoffatomen, substituiert sein.

Es können z.B. folgende aromatische Amine als Ausgangsstoffe II verwendet werden: unsubstituiertes oder in 2-Stellung oder 3-Stellung einfach oder in 2,3-, 2,5-, 3,5-Stellung gleich oder unterschiedlich 2fach durch Chlor, Brom, Methyl, Ethyl-, Propyl-, Isopropyl-, Butyl-, Isobutyl-, sek. Butyl-, tert. Butylgruppen substituiertes Anilin; bevorzugt sind Anilin, o-Toluidin, o-Chloranilin, m-Chloranilin oder 2,5-Xylidin.

Die Umsetzung wird im allgemeinen bei einer Temperatur von 50 bis 150, vorzugsweise von 80 bis 150°C, insbesondere von 90 bis 130°C, mit Unterdruck, Überdruck oder drucklos, diskontinuierlich oder kontinuierlich durchgeführt.

Als Katalysatoren werden organische, sulfonsaure Kationenaustauscher aus Harzen aus sulfoniertem Polystyrol-divinylbenzol verwendet. Es kommen sulfonierte Polystyrol-divinylbenzolaustauscher mit einem vernetzten Anteil zwischen 4 und 14, insbesondere von 4 bis 10 Gew.-% Divinylbenzol, bezogen auf das Copolymerisat, in Frage. Die Austauscher liegen in der Säureform und nicht als Salz vor. Der Katalysator hat eine Korngrösse von 10 bis 200, vorzugsweise von 20 bis 180, insbesondere von 25 bis 150 Mikrometer. Er hat makroretikulare oder in der Regel zweckmässig gelartige Struktur. Der Katalysator kann sowohl im feuchten Zustand als auch wasserfreiem Zustand eingesetzt werden, wobei die Entwässerung auf übliche Weise, z.B. durch Erhitzen auf 100°C im Vakuum, durch Verdrängen mit hydrophilen organischen Flüssigkeiten und anschliessendem Erhitzen auf 100°C bei vermindertem Druck oder durch azeotrope Destillation mit einer organischen Flüssigkeit erfolgen kann. Der Katalysator hat eine spezifische Oberfläche von 2 bis 40, insbesondere von 2 bis 20 Quadratmeter/Gramm Copolymerisat und eine Porenweite von 0,5 bis 40, insbesondere 0,5 bis 20 Nanometern. Unter Porenweite wird der durchschnittliche Durchmesser aller Poren verstanden. Sie werden nach bekannten Methoden durch Mischpolymerisation von Styrol mit Divinylbenzol, Sulfonierung mit Schwefelsäure bzw. Oleum und anschliessender Zerkleinerung hergestellt. Bei kontinuierlicher Arbeitsweise wird die Katalysatormenge allgemein so gewählt, dass der Katalysatorfaktor 1 bis 20, vorzugsweise 1 bis 10 beträgt. Der Katalysatorfaktor ist definiert als

$$\frac{\text{trockener Katalysator (g)}}{\text{Beschickungsgeschwindigkeit (g/Stunde)}}.$$

Die Reaktion kann wie folgt durchgeführt werden: Ein Gemisch von Ausgangsstoff II, Formaldehyd und Katalysator wird bei der Reaktionstemperatur gehalten. Die Reaktionszeit bzw. Verweilzeit beträgt im allgemeinen 3 bis 30, vorzugsweise 5 bis 20 Stunden. Die Umsetzung wird zweckmässig so durchgeführt, dass die wässrige Formaldehydlösung und das aromatische Amin vereinigt werden und der Katalysator zugegeben wird oder aber Anilin bzw. Formaldehyd und der Katalysator vorgelegt werden und dann der Formaldehyd oder die Formaldehyd abgebende Verbindung bzw. Anilin zugeführt wird. Die Zugabe von Anilin bzw. Formaldehyd kann in einer oder mehreren Portionen erfolgen. Nach der Reaktion wird der Endstoff aus dem Reaktionsgemisch in üblicher Weise, z.B. durch Filtration des Katalysators und Destillation, abgetrennt. Das niedriger siedende überschüssige Amin wird über Kopf abdestilliert und in den Prozess zurückgeführt, während das als Destillationsrückstand vorliegende Polyamingemisch unmittelbar, z.B. durch Phosgenierung, in bekannter Weise zu einem Polyisocyanatgemisch weiter verarbeitet werden kann.

Die nach dem Verfahren der Erfindung herstellbaren 4,4'-Diaminodiphenylmethane I sind wertvolle Ausgangsstoffe für die Herstellung von Farbstoffen, Schädlingsbekämpfungsmitteln und insbesondere Kunststoffen. Bezüglich der Verwendung wird auf die vorgenannten Veröffentlichungen und Ullmanns Encyklopädie der technischen Chemie, Band 13, Seiten 339 bis 363 (3. Ausgabe), verwiesen.

Die in den folgenden Beispielen aufgeführten Teile bedeuten Gewichtsteile.

Beispiel 1

Ein Gemisch aus 372 Teilen Anilin, 100 Teilen einer 30 gew.-%igen, wässrigen Formaldehydlösung und 40 Teilen eines pulverförmigen, trockenen gelartigen, mit 8 Gew.-% Divinylbenzol vernetzten Ionentauschers, bestehend aus einem sulfonierten Styrol-divinylbenzol-copolymerisat, wird während 0,5 Stunden unter Rühren auf 100°C erhitzt und 20 Stunden bei 100°C gehalten. Die spezifische Oberfläche beträgt 5 qm/g Katalysator und die mittlere Porenweite 1 Nanometer. Nach Beendigung der Umsetzung wird der Katalysator durch Filtration abgetrennt und nicht umgesetztes Anilin vom Reaktionsgemisch abdestilliert. Man erhält (durch Hochdruckflüssigkeitschromatographie geprüft) 175 Teile Polyamingemisch mit 72 Gew.-% Gesamtdiaminodiphenylmethangehalt oder 94 Gew.-% 4,4'-Diaminodiphenylmethan, 1 Gew.-% 2,2'-Diaminodiphenylmethan und 5 Gew.-% 2,4'-Diaminodiphenylmethan, bezogen auf die Gesamtmenge an Diaminodiphenylmethanen. Bei der Destillation des Polyamingemisches erhält man 4,4'-Diaminodiphenylmethan vom Kp 232–233°C (9 mbar).

Beispiel 2

Ein Gemisch aus 372 Teilen Anilin, 50 Teilen einer 30 gew.-%igen wässrigen Formaldehydlösung und 40 Teilen Katalysator entsprechend Beispiel 1 wird unter Rühren innerhalb von 3 Stunden auf 110°C erhitzt und 17 Stunden bei dieser Temperatur gehalten. Die spezifische Oberfläche beträgt 5 qm/g Katalysator und die Porenweite 1 Nanometer. Das Wasser der Formaldehydlösung und das gebildete Kondensationswasser werden kontinuierlich destillativ abgetrennt. Nach Beendigung der Reaktion wird der Katalysator abgetrennt und nicht umgesetztes Anilin abdestilliert. Man erhält (durch Hochdruckflüssigkeitschromatographie geprüft) 93 Teile Polyamingemisch mit 83 Gew.-% Gesamtdiaminodiphenylmethangehalt oder 92 Gew.-% 4,4'-Diaminodiphenylmethan, 1 Gew.-% 2,2'-Diaminodiphenylmethan und 7 Gew.-% 2,4'Diaminodiphenylmethan, bezogen auf die Gesamtmenge an Diaminodiphenylmethanen.

Beispiel 3

Analog Beispiel 1 wird die Umsetzung mit einem pulverförmigen, gelartigen, trockenen, mit 4,2 % Divinylbenzol vernetztem Austauscher durchgeführt.

Die spezifische Oberfläche des Katalysators beträgt 8 qm/g Katalysator und die Porenweite 1 Nanometer. Man erhält (durch Hochdruckflüssigkeitschromatographie geprüft ) 171 Teile Polyamingemisch mit 70 Gew.-% Gesamtdiaminodiphenylmethangehalt oder 91 Gew.-% 4,4'-Diaminodiphenylmethan, 1 Gew.-% 2,2'-Diaminodiphenylmethan und 8 Gew.-% 2,4'-Diaminodiphenylmethan, bezogen auf die Gesamtmenge an Diaminodiphenylmethanen.

Beispiel 4

Analog Beispiel 1 wird die Umsetzung durchgeführt, wobei nach Beendigung der Umsetzung die Reaktionslösung abgetrennt wird und Anilin und wässriger Formaldehyd in Gegenwart desselben Katalysators erneut zur Reaktion gebracht wird. Nach 10 Wiederholungen der Umsetzung wird die Reaktionslösung nach der destillativen Abtrennung des nicht umgesetzten Anilins mittels Hochdruckflüssigkeitschromatographie geprüft.

Man erhält in alle 11 Umsetzungen jeweils 169 Teile Polyamingemisch mit 70 Gew.-% Gesamtdiaminodiphenylmethangehalt oder 92 Gew.-% 4,4'-Diaminodiphenylmethan, 1 Gew.-% 2,2'-Diaminodiphenylmethan und 7 Gew.-% 2,4'-Diaminodiphenylmethan, bezogen auf die Gesamtmenge an Diaminodiphenylmethanen.

## Patentansprüche

1. Verfahren zur Herstellung von Polyamingemischen mit einem Anteil von mindestens 50 Gew.-% Diaminodiphenylmethanen, und, bezogen auf die Gesamtmenge an Diaminodiphenylmethanen, einem Anteil von mindestens 90 Gew.-% 4,4-Diaminodiphenylmethan der Formel I

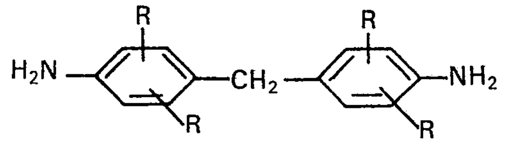

I,

worin die einzelnen Reste R gleich oder verschieden sein können und jeweils ein Wasserstoffatom, ein Halogenatom oder einen aliphatischen Rest bezeichnen, bezogen auf die Gesamtmenge an Diaminodiphenylmethanen, durch Umsetzung von aromatischen Aminen mit Formaldehyd in Gegenwart von sulfonsauren Kationenaustauschern auf Basis Styrol-divinylbenzol-copolymerisaten, dadurch gekennzeichnet, dass man aromatische Amine der Formel II

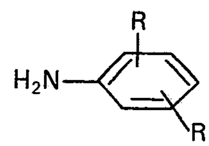

II,

worin R die vorgenannte Bedeutung besitzt, in Gegenwart von Styrol-divinylbenzol-copolymerisaten mit einem vernetzten Anteil zwischen 4 und 14 Gew.-% Divinylbenzol, bezogen auf das Copolymerisat, einer Korngrösse von 10 bis 200 Mikrometer, mit einer spezifischen Oberfläche von 2 bis 40 Quadratmeter/Gramm und mit einer Porenweite von 0,5 bis 40 Nanometern, umsetzt.

2. Verfahren nach Anspruch 1, dadurch gekennzeichnet, dass die Umsetzung in Gegenwart von Wasser durchgeführt wird.

## Revendications

1. Procédé pour la préparation de mélanges de polyamines, contenant une proportion d'au moins 50% en poids de diaminodiphénylméthanes et, par rapport à la quantité totale de diaminodiphénylméthanes, une proportion d'au moins 90% en poids de 4,4'-diaminodiphénylméthane de formule I

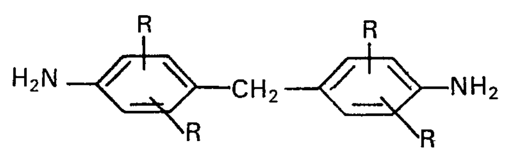

I,

dans laquelle les différents radicaux R peuvent être semblables ou dissemblables et représentent chacun un atome d'hydrogène, un atome d'halogène ou un radical aliphatique, par réaction d'amines aromatiques avec l'aldéhyde formique en présence d'échangeurs de cations sulfonés à base de copolymères de styrène-divinylbenzène, caractérisé en ce qu'on fait réagir des amines aromatiques de formule II

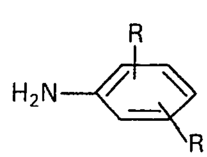

II,

dans laquelle R a la signification donnée ci-dessus, en présence de copolymères de styrène-divinylbenzène qui contiennent, en liaison réticulée, entre 4 et 14% en poids de divinylbenzène par rapport au copolymère, et qui ont une grosseur de grains de 10 à 200 microns, une surface spécifique de 2 à 40 $m^2/g$ et une largeur de pores de 0,5 à 40 nm.

2. Procédé selon la revendication 1, caractérisé en ce que la réaction est menée en présence d'eau.

## Claims

1. A process for the preparation of a polyamine mixture which contains not less than 50% by weight of diaminodiphenylmethanes, which in turn contain not less than 90% by weight of 4,4'-diaminodiphenylmethane of the formula I

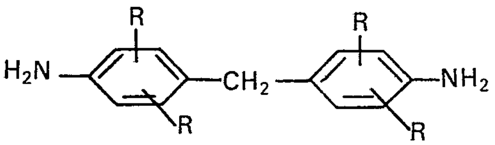

I,

where the R's may be identical or different and each is hydrogen, halogen or an aliphatic radical, by reacting an aromatic amine with formaldehyde in the presence of a cation exchanger based on a styrene/divinylbenzene copolymer and possessing sulfonic acid groups, wherein an aromatic amine of the formula II

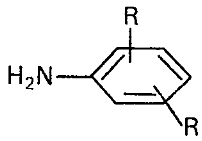

II,

where R has the above meanings, is reacted in the presence of a styrene/divinylbenzene copolymer which is crosslinked with from 4 to 14% by weight, based on the copolymer, of divinylbenzene and has a particle size of from 10 to 200 micrometers, a specific surface area of from 2 to 40 square meters/gram and a pore with of from 0,5 to 40 nanometers.

2. A process as claimed in claim 1, wherein the reaction is carried out in the presence of water.